# EUROPEAN PATENT APPLICATION

(11) **EP 2 609 884 A1**
(43) Date of publication of application: **03.07.2013**
(21) Application number: 12172146.8
(22) Date of filing: 15.06.2012
(51) Int. Cl.: A61B 18/14

(54) **Balloon catheter**

(30) Priority: 26.12.2011 JP 2011283655
(71) Applicant: Japan Electel Inc, Kanagawa 220-0021 (JP)
(72) Inventor: Satake, Shutaro, Kanagawa, 2480031 (JP)
(74) Representative: Brooks, Nigel Samuel

(57) **Abstract**

Thermal conduction to a non-target tissue is unfailingly blocked off to select and effectively ablate only a target tissue. A catheter shaft 1 includes an outer, intermediary and inner tubes 2, 3, 4 which are slidable to each other. An outer balloon 5 is arranged between distal ends 2A, 4A of the outer and inner tubes 4, 5, while an inner balloon 6 is arranged between distal ends 3A, 4A of the intermediary and inner tube 4. The outer balloon 5 is filled with a low thermal conductive gas. Filling solution filled inside the inner balloon 6 is subjected to radiofrequency heating by supplying electricity to an electrode. The outer balloon 5 is held inside a luminal organ and the thermal conduction from the inner balloon 6 is blocked off, thus the thermal energy of the inner balloon 6 selectively ablates a target tissue via a close-contact portion 14.

## Description

### BACKGROUND OF THE INVENTION

### Field of the invention

The present invention relates to a balloon catheter for radiofrequency thermal therapy, which is inserted into a luminal organ to selectively ablate a target tissue such as a site of lesion at optimal temperature.

### Description of the Related Art

The conventional radiofrequency thermal balloon catheter includes a balloon provided with an electrode for delivery of a radiofrequency current therein. The radiofrequency current is applied to the electrode for delivery of radiofrequency current and thus a solution inside of the balloon is heated to raise a surface temperature of the balloon and then the tissue in contact with the balloon is directly ablated. When inserting the balloon catheter into a luminal organ such as a blood vessel, an esophagus, or a large intestine to treat them, it was common for the tissue in contact with the balloon to be ablated circumferentially. This ablation, however, has a risk of causing a cicatricial contraction on the entire inner circumference of the luminal organ and a functional incompetence due to its stenosis.

Therefore, as a method for selectively ablating only a lesion of a luminal organ, it was once proposed to block off thermal conduction to a non-target tissue by thickening a part of a balloon film that is not to come in contact with the target tissue or attaching an insulating material to the part of the balloon film, as disclosed in e.g., Japanese Patent Publication No. 4702704, filed by the present applicant.

According to the balloon catheter employing the method described above, however, it is difficult to retain the balloon at the target tissue and the insulating effect is insufficient, resulting in difficulty in selecting only the target tissue and effectively ablating the same.

### SUMMARY OF THE INVENTION

With a view to the problem described above, it is, therefore, an object of the present invention to provide a balloon catheter capable of blocking off thermal conduction to a non-target tissue to select only a target tissue and then effectively ablate the same.

In order to attain the above object, a first aspect of the present invention is a balloon catheter including a catheter shaft including an outer tube, an intermediary tube and an inner tube which are slidable to each other; an outer balloon arranged between a distal end of the outer tube and a distal end of the inner tube; an inner balloon which is arranged between a distal end of the intermediary tube and the distal end of the inner tube and is housed inside the outer balloon; an electrode and a temperature sensor which are installed inside the inner balloon; a solution transport path formed between the intermediary tube and the inner tube, in communication with an inside of the inner balloon; a gas transport path formed between the outer tube and the intermediary tube, in communication with a portion between the outer balloon and the inner balloon; a radiofrequency generator for supplying electricity to the electrode while measuring the temperature inside the inner balloon via a lead wire of the temperature sensor; a solution injector for transporting a filling solution to the inside of the inner balloon via the solution transport path; a vibration generator for transmitting vibrational waves to the inside of the inner balloon via the solution transport path; and a gas injector for transporting a gas to the portion between the outer balloon and the inner balloon via the gas transport path, wherein there is formed a close-contact portion in which the inner balloon and the outer balloon are allowed to partially come into contact with each other when they are inflated.

A second aspect of the present invention is the balloon catheter, wherein an inner-tube coupling opening provided on a distal end of the inner balloon and an intermediary-tube coupling opening provided on a proximal end of the inner balloon are arranged such that a straight line connecting these opposite tube coupling openings coincides with a central line of the inner balloon; and
an inner-tube coupling opening provided on a distal end of the outer balloon and an outer-tube coupling opening provided on proximal end of the outer balloon are arranged such that a straight line connecting these opposite tube coupling openings is deviated toward one side from a central line of the outer balloon, with the close-contact portion being formed on the one side.

A third aspect of the present invention is the balloon catheter according to the first aspect, wherein an inner-tube coupling opening provided on a distal end of the inner balloon and an intermediary-tube coupling opening provided on proximal end of the inner balloon, as well as an inner-tube coupling opening provided on a distal end of the outer balloon and an outer-tube coupling opening provided on proximal end of the outer balloon, are, respectively, arranged such that a straight line connecting the opposite tube coupling openings coincides with a central line of the respective balloon, whereby the close-contact portion is allowed to be formed by changing shapes and setting positions of the inner and outer balloons on the catheter shaft.

A fourth aspect of the present invention is the balloon catheter according to any one of the first to third aspects, wherein the outer and the inner balloons are made up of a material rich in elasticity.

A fifth aspect of the present invention is the balloon catheter according to any one of the first to third aspects, wherein the outer and inner balloons are made up of a heat resistant resin.

A sixth aspect of the present invention is the balloon catheter according to any one of the first to third aspects, wherein the outer and inner balloons are formed of a body of rotation.

A seventh aspect of the present invention is the balloon catheter according to the first or second aspect, wherein parts of the outer and inner balloons are joined by an adhesive agent.

An eighth aspect of the present invention is the balloon catheter according to the first or second aspect, wherein parts of the inner and outer balloons are joined by being melted.

A ninth aspect of the present invention is the balloon catheter according to the third aspect, wherein the outer and inner balloons are different in shape such that the close-contact portion may be formed by this difference in shape.

A tenth aspect of the present invention is the balloon catheter according to the third aspect, wherein on a distal end side of said close-contact portion, there is formed a gas space between the outer balloon and the inner balloon in a closed manner.

According to the first aspect of the present invention, by the outer balloon filled with a low thermal conductive gas and the inner balloon located inside the outer balloon, in which filling solution filled therein is subjected to radiofrequency heating by supplying electricity to the electrode, the outer balloon inflated is held inside a luminal organ and blocks off the thermal conduction from the inner balloon and then the thermal energy of the inner balloon selectively ablates a target tissue via the close-contact portion between the inner and outer balloons. Therefore, the thermal conduction to a non-target tissue can be reliably blocked off and thus only a target tissue can be selected and effectively ablated.

According to the second aspect of the present invention, since the straight line connecting the tube coupling openings of the outer balloon inserted by the inner tube is deviated toward one side from the central line of the outer balloon, the partial close-contact portion opposed to the target tissue is easily formed on the one side and a space for blocking off the thermal conduction from the inner balloon can be widely-ensured between the outer and inner balloons in the side opposed to the close-contact portion.

According to the third aspect of the present invention, the outer and inner balloons can be arranged on the same central line with respect to the inner tube and a space for blocking off the thermal conduction from the inner balloon can be widely-ensured between the outer and the inner balloons in the side opposed to the close-contact portion with respect to the central line of the outer balloon.

According to the fourth aspect of the present invention, when inflating the outer and the inner balloons, the balloons are easily elastically-deformed by the pressing force from the luminal organ. Therefore, the outer balloon inflated can be held at a given position and besides the target tissue can be reliably pressed against the close-contact portion.

According to the fifth aspect of the present invention, the outer and inner balloons are made of heat resistant resin and thus the trouble such as the thermal deformations of the outer and inner balloons resulting from the thermal energy from the inner balloon can be avoided.

According to the sixth aspect of the present invention, the outer and inner balloons can be formed into various shapes of body of rotation.

According to the seventh aspect of the present invention, the gas and the filling solution can be prevented from leaking from a portion where the outer and inner balloons are adhered together.

According to the eighth aspect of the present invention, the outer and inner balloons are partially melted to be joined, and thus the gas and the filled solution can be prevented from leaking.

According to the ninth aspect of the present invention, the close-contact portion can be formed at a given position by the difference in the shapes of the outer and inner balloons. A space for blocking off the thermal conduction from the inner balloon can be widely-ensured between the outer balloon and the inner balloon except for the position of this close-contact portion.

According to the tenth aspect of the present invention, even if the balloon catheter receives a flow such as a blood flow from the distal end side thereof in a luminal organ, since the gas space is located anterior to the close-contact portion, a thermal energy in the inner balloon can be prevented from being taken away by the flow, and thus efficient ablating of the target tissue can be achieved.

### BRIEF DESCRIPTION OF THE DRAWINGS

FIG. 1 is an explanatory drawing illustrating an overall structure of a balloon catheter and a usage state thereof according to a first embodiment of the present invention.
FIG. 2 is a cross-sectional view on an A-A line of FIG. 1 according to the first embodiment of the present invention.
FIG. 3 is an explanatory drawing illustrating a structure of a main body of the balloon catheter and a usage state thereof according to a second embodiment of the present invention.
FIG. 4 is an explanatory drawing illustrating a structure of a main body of the balloon catheter and a usage state thereof according to a third embodiment of the present invention.
FIG. 5 is an explanatory drawing illustrating a structure of a main body of the balloon catheter and a usage state thereof according to a fourth embodiment of the present invention.

### DESCRIPTION OF THE PREFERRED EMBODIMENTS

Embodiments of a balloon catheter of the present invention are described hereunder with reference to the accompanying drawings.

### First embodiment

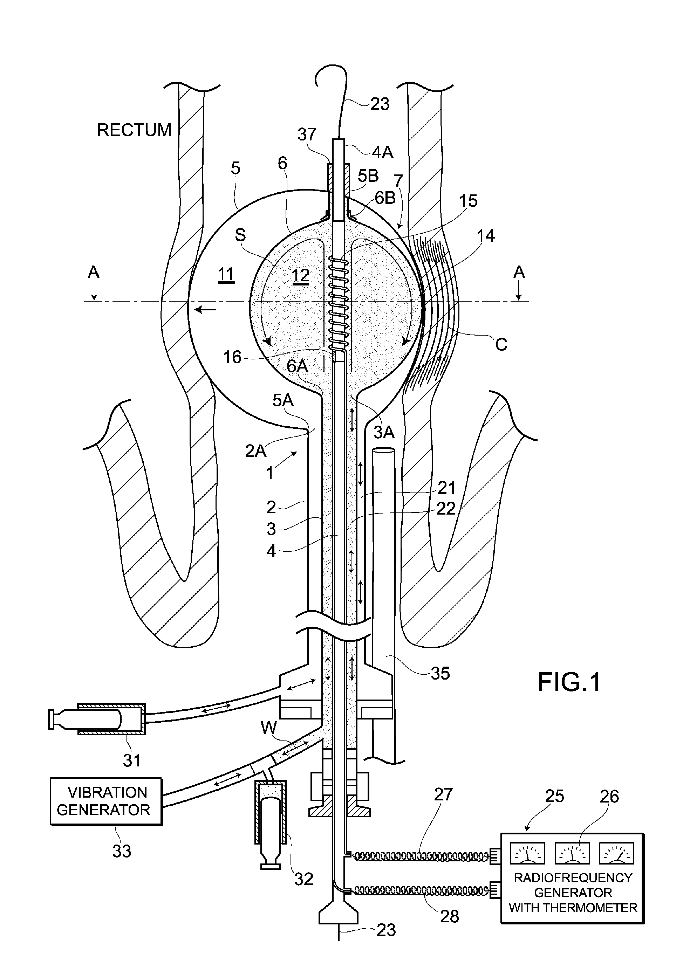

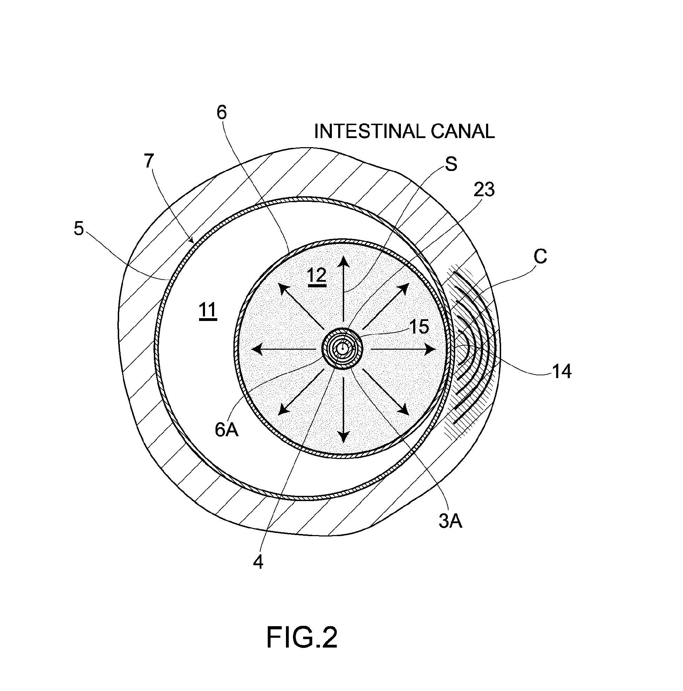

FIG.1 and FIG.2 show a balloon catheter in a first embodiment of the present invention. In these figures, numeral symbol 1 denotes a cylindrical catheter shaft which is insertable into a luminal organ and is richly flexible. The catheter shaft 1 includes an outer tube 2, an intermediary tube 3 and an inner tube 4, which are slidable to each other. An outer balloon 5 including an outer surface exposed to the outside is provided between a distal end 2A of the outer tube 2 and a vicinity of a distal end 4A of the inner tube 4. An inner balloon 6 whose outer surface is surrounded with the outer balloon 5 is provided between a distal end 3A of the intermediary tube 3 and the vicinity of the distal end 4A of the inner tube 4. A balloon part 7 including the structure of a balloon inside a balloon, that is a duplex structure of the outer and inner balloons 5, 6, is provided on the distal end side of the catheter shaft 1.

An internal space between an inner surface of the outer balloon 5 and an outer surface of the inner balloon 6 is formed as a filling part 11 to be filled with a low thermal conductive gas (air or carbon dioxide). Further, an internal space surrounded with the inner balloon 6 is formed as a filling part 12 to be filled with an electrolyte solution and be isolated from the filling parts 11. When the filling part 11 is filled with a gas, the outer balloon 5 inflates in a substantially spherical shape, and when the filling part 12 is filled with a filling solution, the inner balloon 6 inflates in a substantially spherical shape.

The tube coupling openings 5A, 5B inserted by the inner tube 4 are provided on a proximal end side that is a front side of the outer balloon 5 and on a distal end side that is a rear side thereof, respectively, while the tube coupling openings 6A, 6B inserted by the inner tube 4 are provided on a proximal end that is a front side of the inner balloon 6 and on a distal end side that is a rear side thereof, respectively. The tube coupling opening 5A of the outer balloon 5 is near the distal end 2A of the outer tube 2, while the tube coupling opening 6A of the inner balloon 6 is near a distal end 3A of the intermediary tube 3. The distal end 4A of the inner tube 4 is fixed to the tube coupling opening 5B on the distal end side of the outer balloon 5 and the tube coupling opening 6B on the distal end side of the inner balloon 6 and thus the inner tube 4 is allowed to be axially slidable in relation to the outer tube 2 and the intermediary tube 3.

In the balloon part 7 in the present embodiment, a straight line connecting the tube coupling openings 6A, 6B of the inner balloon 6 is provided so as to coincide with a central line acting as a rotation axis of the inner balloon 6, while a straight line connecting the tube coupling openings 5A, 5B of the outer balloon 5 is provided so as to be deviated from a central line acting as a rotation axis of the outer balloon 5. Here, since both the straight line connecting the tube coupling openings 6A, 6B of the inner balloon 6 and the straight line connecting the tube coupling openings 5A, 5B of the outer balloon 5 coincide with an axis line of the inner tube 4, the inner tube 4 is arranged at the center of the inner balloon 6 and at the same time the inner tube 4 is arranged so as to be deviated from the center of the outer balloon 5 to one side thereof. Further, with the outer and inner balloons 5, 6 inflated, in a portion deviated toward one side from the balloon part 7 in contact with a luminal organ, a close-contact portion 14 is formed so that the outer and inner balloons 5, 6 can be partially contact with each other.

In the central area of the inner balloon 6 inside the balloon part 7, an electrode 15 for delivery of radiofrequency current acing as an electrode for heating the inside of the inner balloon 6 is wound around the inner tube 4 in a coiled manner. The electrode 15 for delivery of radiofrequency current has a monopolar structure and is constituted in a manner capable of delivering a radiofrequency current between itself and a counter electrode, not shown, provided outside the catheter shaft 1. By delivering a radiofrequency current, the electrode 15 for delivery of radiofrequency current can produce heat. In addition, the electrode 15 for delivery of radiofrequency current may be formed into a bipolar structure to deliver a radiofrequency current between the two electrodes.

Further, inside the balloon part 7, a temperature sensor 16 contacting with the electrode 15 for delivery of radiofrequency current to detect the temperature thereof is fixed to the proximal end side of the inner tube 4. In the meantime, not shown in the figures in the present embodiment, in addition to the temperature sensor 16, another temperature sensor for detecting the inside temperature of the balloon part 7 may be fixed in the vicinity of the distal end 4A of the inner tube 4. For details of such another temperature sensor, refer to International Application Publication No. 2010/070766 filed by the present applicant.

A gas transport path 21 is formed between the outer and intermediary tubes 2, 3 to transport a low thermal conductive gas to this filling part 11, in communicate with the filling part 11 between the outer and inner balloons 5, 6. Aside from this, a solution transport path 22 is formed between the intermediary and inner tubes 3, 4, to transport an electrolyte solution to this filling part 12 and transmit vibrational waves thereto, in communication with the filling part 12 formed inside the inner balloon 6. Numeral symbol 23 denotes a guide wire for guiding the balloon part 7 to a target site and this guide wire 23 is provided through the inner tube 4.

Outside the catheter shaft 1, a radiofrequency generator 25 is provided for supplying radiofrequency energy, being electricity, to the electrode 15 for delivery of radiofrequency current and heating the solution inside the inner balloon 6. The radiofrequency generator 25 is provided with a thermometer 26 for measuring the temperature of the electrode 15 for delivery of radiofrequency current, eventually, the inside temperature of the inner balloon 6 by a detection signal from the temperature sensor 16 and then displaying the temperature. The radiofrequency generator 25 sequentially takes in the temperature information measured by the thermometer 26 to determine radiofrequency energy supplied to the electrode 15 for delivery of radiofrequency current. Further, to realize such a system, the electrode 15 for delivery of radiofrequency current and the radiofrequency generator 25 are electrically connected by a lead wire 27 and the temperature sensor 16 and the thermometer 26 are electrically connected by another lead wire 28. Then, the lead wires 27, 28 are fixed along the inner tube 4 over the entire axial length of the inner tube 4.

Beyond that, outside the catheter shaft 1, there are provided a syringe 31 acting as a gas injector for supplying a gas into the filling part 11 via the gas transport path 21, a syringe 32 acting as a solution injector for supplying solution to the filling part 12 via the solution transport path 22, and a vibration generator 33 for applying asymmetric vibrational waves W to the filling part 12 through the solution transport path 22 to steadily generate swirls S inside the inner balloon 6, respectively. Then, by changing the pressure of the gas supplied into the filling part 11 by the syringe 31, a diameter of the outer balloon 5 is changed, while by changing the pressure of the solution supplied to the filling part 12 by the syringe 32, a diameter of the inner balloon 6 is changed. Further, the swirls S vibrate and agitate the solution inside the inner balloon 6 to hold the inside the inner balloon 6 with uniform temperature.

Numeral symbol 35 denotes an endoscope for visually checking the position of the balloon part 7 in a luminal organ. As widely known, the endoscope 35 includes a flexible pipe mounted with an optical lens at its distal end and, the inside of the luminal organ can be visually observed by a monitor (not shown) arranged outside the catheter shaft 1.

In the meantime, in the present embodiment, the electrode 15 for delivery of radiofrequency current fixed in the vicinity of the distal end 4A of the inner tube 4 is employed to heat the solution inside the inner balloon 6. However, as it suffices to be able to heat the solution inside the balloon 6, this heating device is not limited to any specific one. As an alternative to the electrode 15 for delivery of radiofrequency current and the radiofrequency generator 25, it is possible to use any one of the followings: a set of an ultrasonic heating element and an ultrasonic generator, a set of a laser heating element and a laser generator, a set of a diode heating element and a diode power supply unit, or a set of a nichrome wire heating element and a nichrome wire power supply unit may be available.

Both the outer balloon 5 and the inner balloon 6 are made of a heat resistant resin, which has durability without thermally deforming in heating the solution inside the inner balloon 6. Instead of the spherical shape whose long and short axes are equal as shown in figures, the shapes of the outer balloon 5 and inner balloon 6 may be those of various bodies of rotation such as an oblate spherical shape whose short axis is defined as a rotation axis, a prolate spheroid whose long axis is defined as a rotation axis, or a bale shape. In any of these shape, the balloon is made up of an elastic membrane which easily deforms in close-contacting to an inside wall of a luminal organ.

Parts of the outer balloon 5 and inner balloon 6 are adhered to each other by an adhesive agent 37. Specifically, the tube coupling opening 5B of the outer balloon 5 and the tube coupling opening 6B of the inner balloon 6 are adhered to together with the distal end 4A of the inner tube 4 by the adhesive agent 37, thereby preventing a gas and solution from leaking from this adhered portion. Instead, parts of the outer balloon 5 and the inner balloon 6 may be joined by a melting process. In this case, without applying the adhesive agent 37, the outer balloon 5 and the inner balloon 6 are partially melted, and thus the gas and the solution can be prevented from leaking from the melted portion.

Next is a description of how to use the balloon catheter in the present embodiment. FIG. 1 and FIG. 2 show an example applied to treat rectal cancer.

First, air-bleeding is performed in the lumen of the catheter, specifically, the filling part 11 and the gas transport path 21 communicating therewith, and the filing part 12 and the solution path 22 communicating therewith. Further, the balloon part 7 is deflated, with the distal ends 2A, 3A of the outer and intermediary tubes 2, 3, and the distal end 4A of the inner tube 4 being slid to each other to maximize the distance therebetween.

After observing a rectal run and a position of a cancer lesion C by a contrast-enhanced imaging of the rectum, a distal-end-looped guide wire 23 is inserted into the rectum and the balloon part 7 which has been deflated and stretched in advance, together with the flexible catheter shaft 1, is inserted into the rectum. At the same time, when the balloon part 7 has been allowed to proceed to a target site inside the rectum while observing the image from the endoscope 35 inserted into the rectum by a monitor not shown, the stretched state of the balloon part 7 is released for the syringe 31 to operate and then an air is injected into the filling part 11 inside the outer balloon 5, inflating the outer balloon 5 by half.

Next, another syringe 32 is operated and the mixed filling solution in which physiological saline and an ionic contrast medium are mixed is injected from the syringe 32 to the filling part 12 in the inner balloon 6 through the solution transport path 22, thereby fully inflating the inner balloon 6. In this state, the whole of the balloon part 7 is rotated around the axis line of the inner tube 4, allowing the inner balloon 6 deviated toward one side in relation to the outer balloon 5 to be faced the cancer lesion C. Then, an air is continued to be further injected from the syringe 31 to the filing part 11 until the whole of the outer balloon 5 is closely contacted by the inside wall of the rectum to be held the position thereof by the pressurizing force from the filling part 11, thus inflating the outer balloon 5. Further, the syringe 32 is operated to attach the inner balloon 6 firmly to the cancer lesion C via the outer balloon 5 and regulate a filling solution volume injected to the filling part 12 inside the inner balloon 6. As a result, by the pressurizing force from the filling part 12, the cancer lesion C, being a target site, is allowed to be closely contacted by the close-contact portion 14 where the outer balloon 5 and the inner balloon 6 are closely contacted by each other.

In this way, after positioning of the balloon part 7 and regulating of the air injection volume injected into the filling part 11 and the filling solution volume injected into the filling part 12, the radiofrequency delivery is started from the radiofrequency generator 25 to the electrode 15 for delivery of radiofrequency current, with the central temperature of the inner balloon 6 observed by a monitor of the thermometer 26. At the same time, the vibration generator 33 is operated and feeds a vibration waves W into the inner balloon 6 through the solution transport path 22 to generate the vertical swirls S inside the inner balloon 6 against gravity, agitating the solution inside the inner balloon 6.

Based on the detection signal from the temperature sensor 16, the radiofrequency generator 25 supplies a radiofrequency current for providing energy so that the inside temperature inside the inner balloon 6, measured by the thermometer 26, becomes the temperature set by an operation section (not shown), to the electrode 15 for delivery of radiofrequency current through the lead wire 27. At this time, the delivery time of radiofrequency current to the electrode 15 for delivery of radiofrequency current is equal to that set by the operation section. Thus, while regulating the central temperature of the inner balloon 6 and the delivery time of radiofrequency current to the electrode 15 for delivery of radiofrequency current, the cancer lesion C in contact with the close-contact portion 14 of the balloon part 7 is pressed and ablated.

Specifically, with the central temperature inside the inner balloon 6 kept at 70 degrees C, a radiofrequency current is delivered to the electrode 15 for delivery of radiofrequency current during the time according to the depth of a cancer lesion C based on a preoperative diagnosis. Commonly, in the case that the depth of the cancer lesion C is from 1 to 2mm, the delivery time of a radiofrequency current to the electrode 15 for delivery of radiofrequency current is determined as 1 to 2 minutes, while in the case that the depth of the cancer lesion C is from 2.5 to 3mm, the delivery time of a radiofrequency current to the electrode 15 for delivery of radiofrequency current is determined as 3 to 4 minutes. In the case that the central temperature inside the inner balloon 6 is at 70 degrees C (in this case, the contact temperature is 60 to 65 degrees C) and the delivery time of a radiofrequency current to the electrode 15 for delivery of radiofrequency current is determined as 1 to 2 minutes, since a muscular layer is not deeply ablated, even if the muscular layer is widely ablated, neither stenosis nor deglutition disorder are caused. On the other hand, in the case of the delivery time for 3 to 4 minutes, since the muscular layer is deeply ablated, if the muscular layer is widely ablated, a dysfunction is caused, thus needing to perform the ablation with care. Generally, it is said that when ablating is performed within one third of an entire circumference of a luminal organ, no dysfunction is caused. An endoscopic mucosal resection is not applicable to a lesion reaching a muscular layer. On the other hand, according to the above method of the present embodiment, if no metastasis occurs, the lesion can be treated even if the lesion has reached the muscular layer. At the temperature on the order of 60 degrees C, as long as an anatomical structure is held even if transmural coagulative necrosis has occurred, a substantial portion of all muscular layers of inner membranes can be regenerated if an ablation range is narrow. This method is similarly applicable to lesions of a large intestine except for a rectum, a small intestine and an esophagus.

### Second embodiment

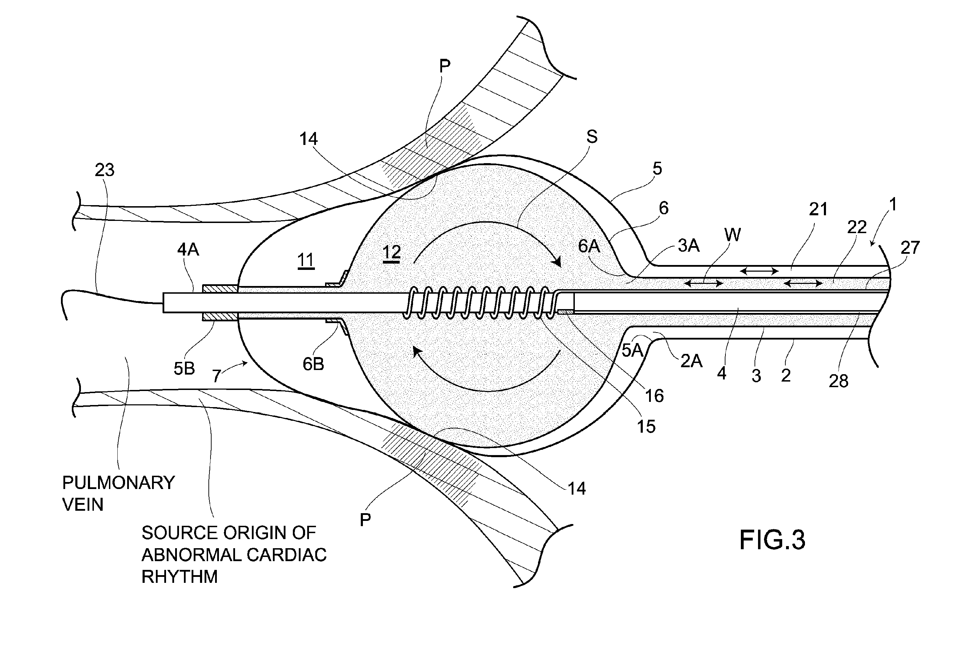

FIG. 3 shows a balloon catheter according to a second embodiment of the present invention. According to the balloon part 7 of the present embodiment, not only a straight line connecting the tube coupling openings 6A, 6B coincides with the central line of the inner balloon 6 inflating into an substantially spherical shape but also a straight line connecting the tube coupling openings 5A, 5B coincides with the central line of the outer balloon 5 inflating into an substantially gourd shape or an substantially bale shape. Further, with the outer and inner balloons 5, 6 inflated, on a circumference in a radial direction of the balloon part 7, a close-contact portion 14 is formed where the outer and inner balloons 5, 6 can be partially closely contacted by each other.

The outer balloon 5 and the inner balloon 6 may be different in shape as in the present embodiment, and further with the outer and inner balloons 5, 6 inflated, when the outer balloon 5 is pressed against the inside wall of a luminal organ, the outer balloon 5 elastically deforms by the pressing force from the inside wall of the luminal organ on the circumference in the radial direction of the balloon part 7 and thus the close-contact portion 14 may be formed. Except for this structure, the structure of the balloon catheter of this embodiment is in common with that in the first embodiment.

Next is a description of how to use the balloon catheter in the present embodiment. FIG. 3 shows an example of the application of the balloon catheter to a pulmonary veins isolation.

The pulmonary veins isolation is effective for treating atrial fibrillation. When you strongly ablate the insides of the pulmonary veins by the balloon catheter, however, a complication of a pulmonary vein stenosis is caused. Then, the balloon part 7 with a balloon-inside-balloon structure is employed as shown in FIG. 3.

Also in this case, first, after performing the air bleeding and deflating of the balloon part 7 as with the first embodiment, the balloon part 7, together with the catheter shaft 1, is inserted from a femoral vein into the pulmonary vein by using a guide sheath (not shown) provided in the periphery of the outer tube 2 and the guide wire 23. Then carbon dioxide is injected from the syringe 31 into the filling part 11 inside the outer balloon 5 through the gas transport path 21, and thus the outer balloon 5 is inflated by carbon dioxide. Next, the electrolyte solution is injected from the syringe 32 into the filling part 12 inside the inner balloon 6 through the solution transport path 22 and thus the inner balloon 6 is inflated by the electrolyte solution, pressing the close-contact portion 14 of the outer and inner balloons 5, 6 against a circumference of a pulmonary vein ostium (P).

By delivering a radiofrequency current from the radiofrequency generator 25 to the electrode 15 for delivery of radiofrequency current, the circumference of a pulmonary vein ostium (P) in contact with the inner balloon 6 via the outer balloon 5 is ablated. Thermal conduction from the inside of the inner balloon 6 is blocked off by carbon dioxide filled in the filling part 11 inside the outer balloon 5 and thus the inside of the pulmonary vein is not ablated. As a result, electrical isolation of the pulmonary vein can be achieved without causing pulmonary vein stenosis. Further, since the thermal energy inside the inner balloon 6 can be prevented from being lost by the bloodstream within the left atrial by the carbon dioxide in the vicinity of the outer balloon 5, the circumference of a pulmonary vein ostium (P) can be effectively ablated.

### Third Embodiment

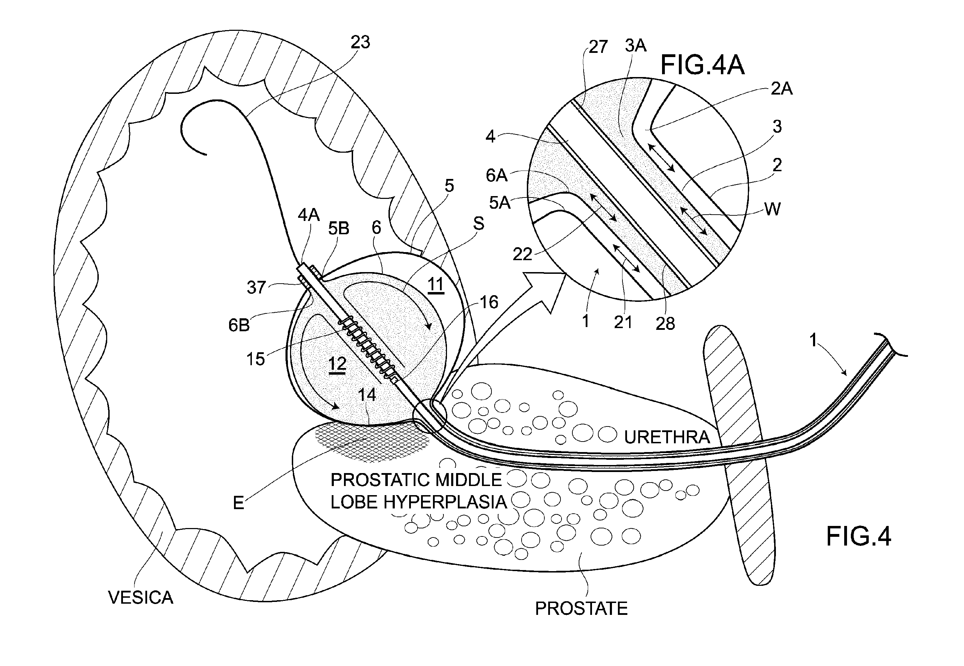

FIG. 4 shows a balloon catheter according to a third embodiment of the present invention. As with the first embodiment, in the balloon part 7 in the present embodiment, a straight line connecting the tube coupling openings 6A, 6B of the inner balloon 6 coincides with the central line of the inner balloon 6, while a straight line connecting the tube coupling openings 5A, 5B is deviated from the central line of the outer balloon 5. Note that, however, whilst the outer balloon 5 is spherical as is the case in the inner balloon 6 in the first embodiment, the inner balloon 6 is spherical and the outer balloon 5 is oblate in form in the present embodiment. Other structures are in common with those in the first embodiment.

Next is a description of how to use the balloon catheter according to the present embodiment. FIG. 4 shows an example of the application of the balloon catheter to a treatment of a hypertrophy of prostate medial lobe.

The conventional devices utilizing microwaves or radiofrequency waves for treating prostate hypertrophy have been unable to ablate a prostate medial lobe though they have been able to ablate a prostate lateral lobe. Therefore, the balloon part 7 with a balloon-inside-balloon structure is employed, as shown in FIG. 4.

First, after performing the air bleeding and deflating of the balloon part 7 as with the first embodiment, the distal-end-looped guide wire 23 is inserted into an urethra so that the balloon part 7 deflated in advance, together with the catheter shaft 1, is inserted into the urethra via this guide wire. When the balloon part 7 has come to the inside of a bladder, air is injected from the syringe 31 into the filling part 11 inside the outer balloon 5 through the gas transport path 21 to inflate the outer balloon 5 and then inject the filling solution from the syringe 32 into the filling part 12 inside the inner balloon 6 through the solution transport path 22 to thereby inflate the inner balloon 6. Then, a radiofrequency current is delivered from the radiofrequency generator 25 to the electrode 15 for delivery of radiofrequency current, thereby making it possible to selectively ablate a hypertrophic area E of the prostrate medial lobe by the inner balloon 6, with the bladder wall protected by the outer balloon 5. Further, when the balloon part 7 is subjected to pressuring and heating, with the balloon catheter inserted into the urethra and the balloon part 7 stayed inside the urethra, the prostate lateral lobes also can be ablated one by one, permitting useless ablation to be avoided.

### Fourth Embodiment

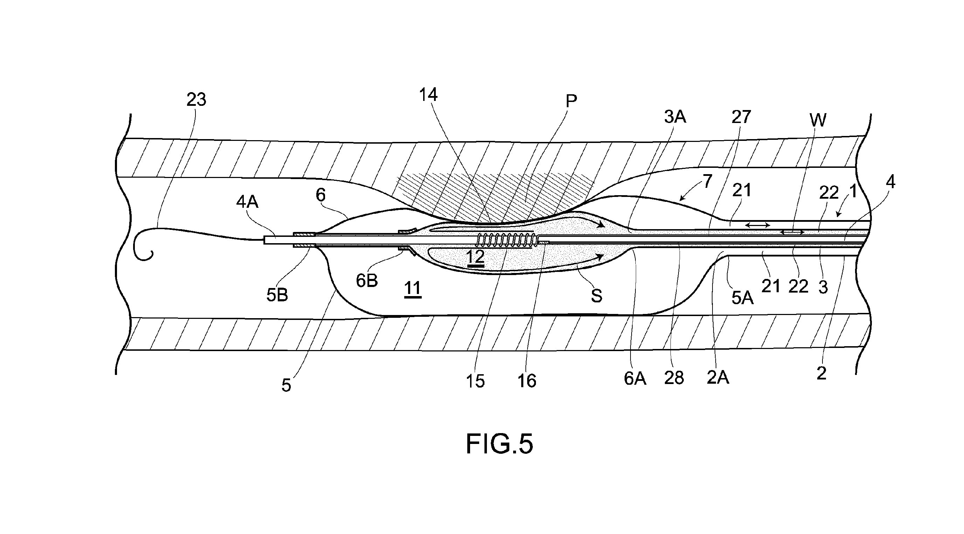

FIG. 5 shows a balloon catheter according to a fourth embodiment in the present embodiment. As with the first embodiment, according to the balloon part 7 of the present embodiment, a straight line connecting the tube coupling openings 6A, 6B of the inner balloon 6 coincides with the central line of the inner balloon 6, while a straight line connecting the tube coupling openings 5A, 5B of the outer balloon 5 is deviated from the central line of the outer balloon 5. The shapes of the outer and inner balloons 5, 6, however, are both prolate-spheroid-shaped. Other structures are in common with those in the first embodiment.

Next is a description of how to use the balloon catheter according to the present embodiment. FIG. 5 shows an example of the application of the balloon catheter to the treatment of eccentric-distributed atherosclerotic plaque P.

When the atherosclerotic plaque is eccentric distributed, the balloon part 7 with a balloon-inside-balloon structure as shown in FIG. 5 is employed. In this case, first, after performing the air bleeding and deflating of the balloon part 7 as with the first embodiment, the distal-end-looped guide wire 23 is inserted into a vein, and the balloon part 7 deflated in advance is inserted into the vein via the guide wire 23, together with catheter shaft 1. When the balloon part 7 has advanced to the vicinity of the atherosclerotic plaque P that is a target tissue, carbon dioxide is injected from the syringe 31 into the filling part 11 inside the outer balloon 5 through the gas transport path 21 to inflate the outer balloon 5, while the filling solution is injected from the syringe 32 into the filling part 12 inside the inner balloon 6 through the solution transport path 22 to thereby inflate the inner balloon 6.

A radiofrequency current is delivered from the radiofrequency generator 25 to the electrode 15 for delivery of radiofrequency current and thus the atherosclerotic plaque P in contact with the inner balloon 6 via the outer balloon 5 is ablated. At this moment, a normal blood vessel wall, however, is not ablated and hyperthermia can be applied only to the atherosclerotic plaque P in contact with the close-contact portion 14 of the balloon part 7. Since the rupture of an atherosclerotic cap is considered to be caused by a lytic enzyme given off by macrophages, the atherosclerotic plaque P becomes stable by destroying the macrophages by the thermotherapy at 43 degrees C. When the atherosclerotic plaque P is pressurized by the balloon part 7 while softening collagen fibers as a main component of a vessel matrix by radiofrequency heating from the balloon part 7, a stenosis site can be dilated under comparatively low pressure without a vascular dissection.

According to the conventional radiofrequency thermal balloon catheter, it is difficult for the balloon part to be selectively brought into contact with a target tissue and therefore the sites in contact with the balloon part are ablated without discrimination, thus damaging also normal tissues. On the contrary, in accordance with the radiofrequency balloon-inside-balloon ablation catheter according to the present invention, the outer balloon 5 is held in position when it is inflated inside a hallow organ and blocks off thermal conduction between itself and the inner balloon 6. At the same time, the inner balloon 6 is selectively brought into contact with a target tissue via the outer balloon 5, and the thermal energy of the inner balloon 6 is transmitted via the close-contact portion 14, allowing the catheter to selectively ablate only the target tissue.

Further, since both the inner balloon 6 and the outer balloon 5 ablate a target tissue with the target tissue pressurized, even if the target tissue is a cancer tissue (e.g., the cancer lesion C in the first embodiment) rich in a blood flow, a sufficient ablating effect can be obtained because the cancer tissue is heated while shutting off the blood flow to reduce the cooling effect caused by the blood flow. Further, since the normal tissue is protected from the ablation due to the outer balloon 5 filled with the low thermal conductive gas, no collateral damage occurs.

As discussed above, while the conventional radiofrequency balloon ablation catheter includes a single balloon structure, the balloon catheter according to the present invention includes the balloon part 7 with a balloon-inside-balloon structure. Specifically, in the radiofrequency thermal balloon according to the present invention, the inner balloon 6 is arranged inside the outer balloon 5 filled with the low thermal conductive gas, and hence, when the inner balloon 6 is contacted by the target tissue via the outer balloon 5, the target tissue is ablated by the thermal conduction from the closely-contacted surface. As a result, only the target tissue can be selectively treated without damaging normal tissues.

As described above, the balloon catheter according to the present invention includes:
the catheter shaft 1 made up of the outer tube 2, the intermediary tube 3 and the inner tube 4 which are slidable to each other;
the outer balloon 5 which is arranged between the distal end 2A of the outer tube 2 and the distal end 4A of the inner tube 4 and is inflatable;
the inner balloon 6 which is arranged between the distal end 3A of the intermediary tube 3 and the distal end 4A of the inner tube 4 and is housed inside the outer balloon 5 and further is inflatable;
the electrode 15 for delivery of radiofrequency current and the temperature sensor 16 which are installed inside the inner balloon 6;
the solution transport path 22 formed between the intermediary tube 3 and the inner tube 4, in communication with the inside of the inner balloon 6;
the gas transport path 21 formed between the outer tube 2 and the intermediary tube 3, in communication with the portion between the outer balloon 5 and the inner balloon 6;
the radiofrequency generator 25 acting as a radiofrequency generating unit for supplying electricity to the electrode while measuring the inside temperature of the inner balloon 6 via the lead wire 28 of the temperature sensor 16;
the syringe 32 for transporting the filling solution to the inside of the inner balloon 6 via the solution transport path 22;
the vibration generator 33 for transmitting vibrational waves to the inside of the inner balloon 6 via the solution transport path 22; and
the syringe 31 for transporting a gas to the portion between the outer balloon 5 and the inner balloon 6 via the gas transport path 21,
wherein the close-contact portion 14 in which the inner balloon 6 and the outer balloon 5 are allowed to partially come into contact with each other when they are inflated is formed in the balloon part 7 including the outer balloon 5 and the inner balloon 6, as the features common to the above first to fourth embodiments.

In this case, due to the outer balloon 5 filled with the low thermal conductive gas and the inner balloon 6 located inside the outer balloon 5, the inner balloon 6 being filled with the filling solution and subjected to radiofrequency heating by supplying electricity to the electrode 15 for delivery of radiofrequency current, the outer balloon 5, when inflated, is allowed to be held in position inside a luminal organ and block off the thermal conduction from the inner balloon 6, and then, the thermal energy of the inner balloon 6 is allowed to selectively ablate a target tissue via the close-contact portion 14 between the inner and outer balloons 5,6. Therefore, the thermal conduction to any non-target tissues can be reliably blocked off and thus only the target tissue can be selected and effectively ablated.

Further, in the first, third and fourth embodiments, the tube coupling opening 6B corresponding to the inner-tube coupling opening on the distal end side of the inner balloon 6 and the tube coupling opening 6A corresponding to the intermediary-tube coupling opening on the proximal end side of the inner balloon 6 are provided so that the straight line connecting these tube coupling openings 6A, 6B coincides with the central line of the inner balloon 6 acting as a rotation axis thereof, whereas the tube coupling opening 5B corresponding to the inner-tube coupling opening on the distal end side of the outer balloon 5 and the tube coupling opening 5A corresponding to the outer-tube coupling opening on the proximal end side are provided so that the straight line connecting these tube coupling openings 5A, 5B is deviated toward one side from the central line acting as the rotation axis of the outer balloon 5. The close-contact portion 14 is formed on the one side thus deviated.

In this way, the straight line connecting the tube coupling openings 5A, 5B of the outer balloon 5 penetrated by the inner tube 4 is deviated toward the one side from the central line of the outer balloon 5, and thus, the close-contact portion 14 opposed to the target tissue can be easily and partially formed on the one side, while the wide space (filling part 11) for blocking off the thermal conduction from the inner balloon 6 can be ensured between the outer and inner balloons 5, 6 on the side opposed to the close-contact portion 14.

Alternatively, according to the second embodiment, the tube coupling openings 6A, 6B of the inner balloon 6 as well as the tube coupling openings 5A, 5B of the outer balloon 5 are provided so that the straight line connecting the respective opposite tube coupling openings 6A, 6B, and 5A, 5B coincide with the central line of the respective inner and outer balloons 5, 6, whereby the close-contact portion 14 is formed by changing shapes and setting positions of the inner and outer balloons 5, 6 on the catheter shaft 1.

In this case, the outer balloon 5 and the inner balloon 6 can be arranged on the same central line with respect to the inner tube 4 and besides by changing the shapes of the outer balloon 5 and inner balloon 6 and the setting position of both the balloons 5, 6 on the catheter balloon 1, the close-contact portion 14 can be easily formed.

In the second embodiment, the outer balloon 5 and the inner balloon 6 may be different in shape and then the close-contact portion 14 may be formed by this difference in shape.

In this case, the close-contact portion 14 can be formed at any position by utilizing the difference in the shapes of the outer balloon 5 and the inner balloon 6. Further, a wide space (the filling part 11) for blocking off the thermal conduction from the inner balloon 6 can be ensured in a portion between the outer balloon 5 and the inner balloon 6 except for where this close-contact portion 14 is provided.

Furthermore, according to the second embodiment, on the distal end side of the close-contact portion 14, there is formed the filling part 11 acting as a gas space between the outer balloon 5 and the inner balloon 6 in a closed manner. As a result, even if the balloon catheter receives a flow such as a blood flow from the distal end side thereof in a luminal organ, since the gas space is located on the distal end side of the close-contact portion 14, a thermal energy in the inner balloon 6 can be prevented from being lost by the flow due to the gas space serving as a heat insulator, and thus efficient ablating of the target tissue can be achieved.

Moreover, in common with the above first to fourth embodiments, the outer balloon 5 and the inner balloon 6 are both made up of heat resistant resin and formed into a shape of body of rotation.

Thus, when inflating the outer balloon 5 and the inner balloon 6, the outer balloon 5 and the inner balloon 6 are easily elastically-deformed by the pressing force from a luminal organ. Therefore, the outer balloon 5 inflated can be held at a given position and a target tissue can be reliably pressed against the close-contact portion 14. Moreover, the outer balloon 5 and the inner balloon 6 are made of heat resistant resin and thus the trouble such as the thermal deformations of the outer balloon 5 and the inner balloon 6 resulting from the thermal energy from the inner balloon 6 can be avoided. Further, the outer balloon 5 and the inner balloon 6 can be formed into various shapes of body of rotation.

Further, in the first or third embodiments, parts of the outer balloon 5 and the inner balloon 6 are joined by the adhesive agent 37.

As a result, the gas and the filling solution can be prevented from leaking from the portion where the outer and inner balloons 5, 6 are joined together.

Instead, the parts of the outer balloon 5 and the inner balloon 6 may be joined by being melted.

Therefore, the outer balloon 5 and the inner balloon 6 are partially melted, and thus the gas and the filled solution can be prevented from leaking.

In the meantime, the present invention is not limited to the above embodiments and various modifications are possible within the scope of the gist of the present invention. For example, the shapes of the outer and inner balloons 5, 6 are not limited to ones shown in the above embodiments and various shapes may be formed according to the area of the body to be treated.

## Claims

1. A balloon catheter comprising:
a catheter shaft made up of an outer tube, an intermediary tube and an inner tube which are slidable to each other;
an outer balloon arranged between a distal end of said outer tube and a distal end of said inner tube;
an inner balloon which is arranged between a distal end of said intermediary tube and the distal end of said inner tube and is housed inside said outer balloon;
an electrode and a temperature sensor which are installed inside said inner balloon;
a solution transport path formed between said intermediary tube and said inner tube, in communication with an inside of said inner balloon;
a gas transport path formed between said intermediary tube and said-outer tube, in communication with a portion between said outer balloon and said inner balloon;
a radiofrequency generator for supplying electricity to said electrode while measuring temperature inside said inner balloon via a lead wire of said temperature sensor;
a solution injector for transporting a filling solution to the inside of said inner balloon via said solution transport path;
a vibration generator for transmitting vibrational waves to the inside of said inner balloon via said solution transport path; and
a gas injector for sending a gas to a portion between said outer balloon and said inner balloon via said gas transport path,
wherein there is formed a close-contact portion in which said inner balloon and said outer balloon are allowed to partially come into contact with each other when they are inflated.

2. The balloon catheter according to claim 1, wherein an inner-tube coupling opening provided on a distal end of said inner balloon and an intermediary-tube coupling opening provided on a proximal end of said inner balloon are arranged such that a straight line connecting these opposite tube coupling openings coincides with a central line of said inner balloon, and
an inner-tube coupling opening provided on a distal end of said outer balloon and an outer-tube coupling opening provided on a proximal end of said outer balloon are arranged such that a straight line connecting these opposite tube coupling openings is deviated toward one side from a central line of said outer balloon, with said close-contact portion being formed on said one side.

3. The balloon catheter according to claim 1, wherein an inner-tube coupling opening provided on a distal end of said inner balloon and an intermediary-tube coupling opening provided on a proximal end of said inner balloon, as well as an inner-tube coupling opening provided on a distal end of said outer balloon and an outer-tube coupling opening provided on a proximal end of said outer balloon, are, respectively, arranged such that a straight line connecting the opposite tube coupling openings coincides with a central line of the respective balloon, whereby said close-contact portion is allowed to be formed by changing shapes and setting positions of said inner and outer balloons on said catheter shaft.

4. The balloon catheter according to any one of claim 1 to claim 3, wherein said outer and inner balloons are made up of a material rich in elasticity.

5. The balloon catheter according to any one of claim 1 to claim 3, wherein said outer and inner balloons are made up of a heat resistant resin.

6. The balloon catheter according to any one of claim 1 to claim 3, wherein said outer and inner balloons are formed of a body of rotation.

7. The balloon catheter according to claim 1 or claim 2, wherein parts of said outer and inner balloons are joined by an adhesive agent.

8. The balloon catheter according to claim 1 or claim 2, wherein parts of said inner and outer balloons are joined by being melted.

9. The balloon catheter according to claim 3, wherein said outer and inner balloons are different in shape such that said close-contact portion may be formed by this difference in shape.

10. The balloon catheter according to claim 3, wherein on a distal end side of said close-contact portion, there is formed a gas space between said outer balloon and said inner balloon in a closed manner.
